# EUROPEAN PATENT APPLICATION

(11) **EP 2 412 340 A1**
(43) Date of publication of application: **01.02.2012**
(21) Application number: 10755792.8
(22) Date of filing: 19.02.2010
(51) Int. Cl.: A61F 5/44, A61F 13/15, A61F 13/49, A61F 13/511, A61F 13/53

(54) **BODILY FLUID TREATMENT MATERIAL AND WEARING ARTICLE INCLUDING SAME**

(30) Priority: 26.03.2009 JP 2009077599; 30.09.2009 JP 2009228680
(71) Applicant: Unicharm Corporation, Ehime 799-0111 (JP)
(72) Inventor: MITSUI, Koichiro, Kanonji-shi Kagawa 769-1602 (JP); AOKI, Katsufumi, Kanonji-shi Kagawa 769-1602 (JP); DETANI, Kou, Kanonji-shi Kagawa 769-1602 (JP)
(74) Representative: Peter, Julian
(86) International application number: PCT/JP2010/052493
(87) International publication number: WO 2010/109991

(57) **Abstract**

The present invention aims to provide a bodily fluid trapping unit assuring that bodily fluid can be guided into a receptacle without anxiety of flowing back and a wearing article including such bodily fluid trapping unit. A receptacle 10 of a bodily fluid trapping unit 1 is defined by first and second sheets 12, 13 bonded together along respective peripheral edges thereof so as to form a bag. The first sheet 12 is formed with openings 11 extending therethrough in its thickness direction. Backflow preventing means 20 includes a flat valve disc 30 provided on the side of the first sheet 12 facing the receptacle 10, a sheet-like support base 40 serving to prevent the valve disc 30 from falling into the receptacle 10 and a spacer 50 interposed between the first sheet 12 and the support base 40. A peripheral edge of the valve disc's upper surface 31 is adapted to come in water-tight contact with the associated valve seat 14 formed on the side of the opening's peripheral edge facing the receptacle 10. Between the first sheet 12 and the support base 40, a space is defined by a spacer's opening 53 so that the valve disc 30 may be movably supported in the thickness direction of the spacer 50.

## Description

### {Technical Field}

The present invention relates to bodily fluid trapping units and wearing articles including the same and more particularly to wearing articles such as disposable diapers, toilet-training pants or incontinent briefs respectively including bodily fluid trapping units.

### {Background}

Conventionally, Units adapted to trap body wastes and guide it into the receptacle is known, for example, from JP 4059374 B2 (PTL 1). A disposable diaper disclosed in PTL 1 basically includes a top-sheet, a back-sheet and an absorbent structure sandwiched between these top- and back-sheets. The top-sheet of the diaper is formed with a plurality of partially opened gates through which body waste such as loose passage flows toward the absorbent structure. Such body waste such as loose passage flowing through these partial opened gates is then absorbed by the absorbent structure and body waste should not flow on the top-sheet and not eventually leak out from the diaper.

### {Citation List}

### {Patent Literature}

{PTL 1} JP 4059374 B2

### {Summary}

### {Technical Problem}

The partially opened gate as has been described above is not formed by partially cutting out the top-sheet but by partially incising the top-sheet, leaving an adjacent portion not incised so as to serve as a hinge region about which the gate may be opened and shut. With such an arrangement, it is impossible for body waste to pass through the hinge region and, depending on various factors such as a particular position and/or range of the hinge region, a particular spot onto which the wearer urinates or defecates and a particular posture of the wearer, body waste may not pass through the partially opened gates and be left on the top-sheet.
Furthermore, the partially opened gate is defined not by partially cutting out the top-sheet but by partially incising the top-sheet and, with such an arrangement, the gate may be opened not only inward but also outward. This means that, if body waste flows back from the side of the absorbent structure, the partially opened gate may be opened outward and body waste may outflow onto the top-sheet.

An object of the present invention is to provide bodily fluid trapping units ensuring that bodily fluid can be guided into a receptacle without anxiety of flowing back and wearing articles including such bodily fluid trapping units.

### {Solution to Problem}

The present invention includes a first aspect and a second aspect.
According to the first aspect of the present invention, there is provided a bodily fluid trapping unit includes openings through which bodily fluid flows in and a receptacle adapted to collect bodily fluid flowing thereinto.

The first aspect of the invention further includes the bodily fluid trapping unit including backflow preventing means adapted to restrict outflow of bodily fluid once having been collected in the receptacle through the openings and a plurality of the openings penetrating a part of the receptacle in a thickness direction; the backflow preventing means including a valve seat formed along a peripheral edge of the openings, a valve disc adapted to come from the side of the receptacle in water-tight contact with the valve seat and a support base adapted to prevent the valve disc from falling into the receptacle wherein the bodily fluid is allowed to flow into the receptacle as the valve disc is spaced from the valve seat.

According to one or more embodiments of the present invention on the first aspect thereof, a spacer is interposed between the valve seat and the support base to maintain a space and the valve disc is movably supported within the space.

According to one or more embodiments of the present invention on the first aspect thereof, the valve disc is flat and has an area larger than an opening area of each of the openings.

According to one or more embodiments of the present invention on the first aspect thereof, the valve seat is defined by a valve seat body formed with a valve port extending through the valve seat body in its thickness direction and the valve seat body includes a small diameter section having a diameter smaller than that of the valve port, a large diameter section having a diameter larger than that of the valve port and a contact section defined between the small diameter section and the large diameter section and adapted to come in water-tight contact with the valve port wherein the small diameter section lies on the side facing away from the receptacle and the large diameter section lies on the side facing the receptacle.

According to one or more embodiments of the present invention on the first aspect thereof, the valve disc has a specific gravity lower than that of the bodily fluid.

According to one or more embodiments of the present invention on the first aspect thereof, the backflow preventing means includes a valve disc provided so as to overlap along the periphery of the opening and bonding regions in which the valve disc is bonded to the receptacle, each of the bonding regions is defined by a pair of bonding lines extending outward from the opening so that each pair of the adjacent bonding regions defines a channel allowing bodily fluid to flow into the receptacle through the opening.

According to one or more embodiments of the present invention on the first aspect thereof, two or more types of the backflow preventing means having different modes of action.

According to the second aspect of the present invention, there is provided a wearing article having a longitudinal direction and a transverse direction including a skin-facing side, a garment-facing side, a front waist region, a rear waist region and a crotch region extending between these front and rear waist regions.

According to the second aspect of the present invention, the wearing article includes the bodily fluid trapping unit at least in the crotch region and the openings of the bodily fluid trapping unit are formed on the skin-facing side.

### {Advantageous Effects of Invention}

The bodily fluid trapping unit according to the first aspect of the present invention is provided with the backflow preventing means adapted to ensure that discharged bodily fluid flows into the receptacle through the openings of the receptacle and that bodily fluid should not leak out from the receptacle. With this arrangement, it is possible to restrict bodily fluid leakage from the bodily fluid trapping unit. The bodily fluid trapping unit includes the valve disc or valve plug and the valve seat adapted to come in water-tight contact with each other wherein the valve disc or valve plug is provided so as to cooperate with the openings. In this way, undesirable bodily fluid leakage from the receptacle is reliably restricted so far as the valve disc is in water-tight contact with the associated valve seat.

In the wearing article according to the second aspect of the present invention, the bodily fluid trapping unit is provided at least in the crotch region and the opening of the receptacle is formed on the skin-facing side. When the wearing article is used as a diaper, urine discharged from the wearer can be contained within the bodily fluid trapping unit and thereby urine leakage from the wearing article can be reliably restricted.

### {Brief Description of Drawings}

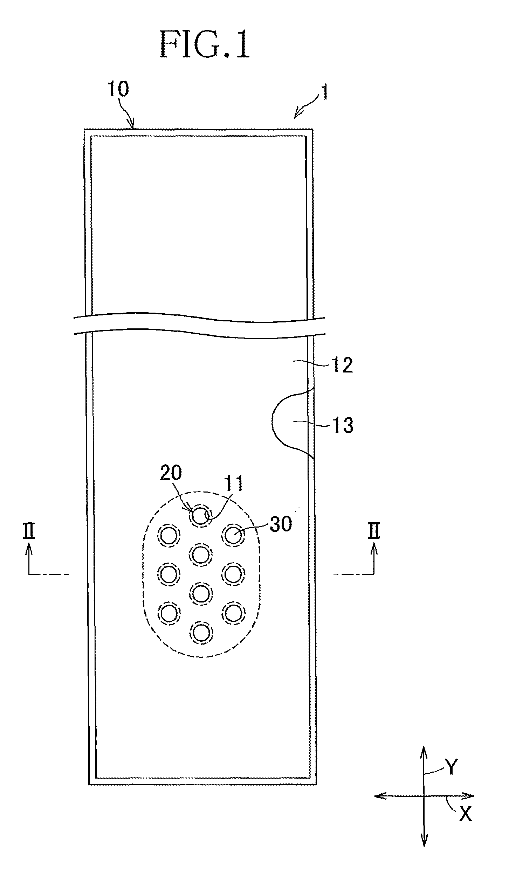

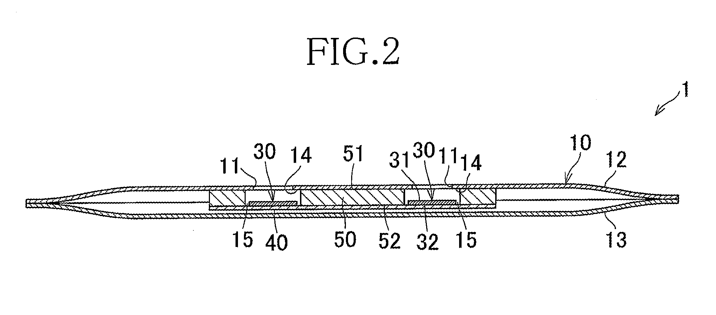

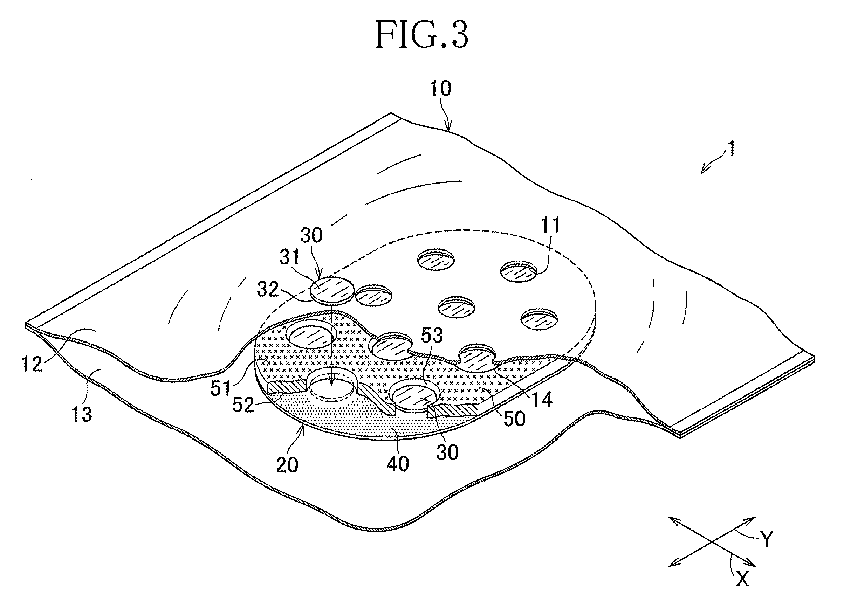

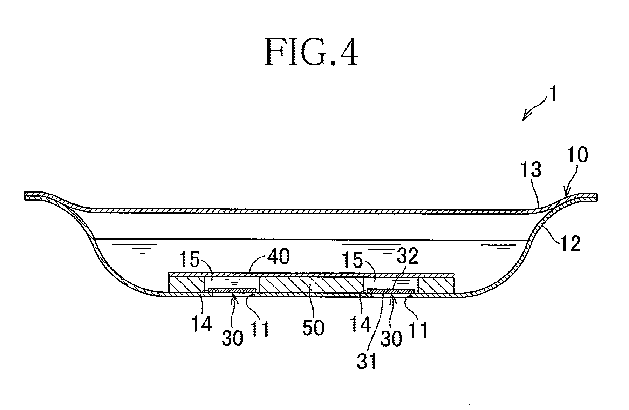

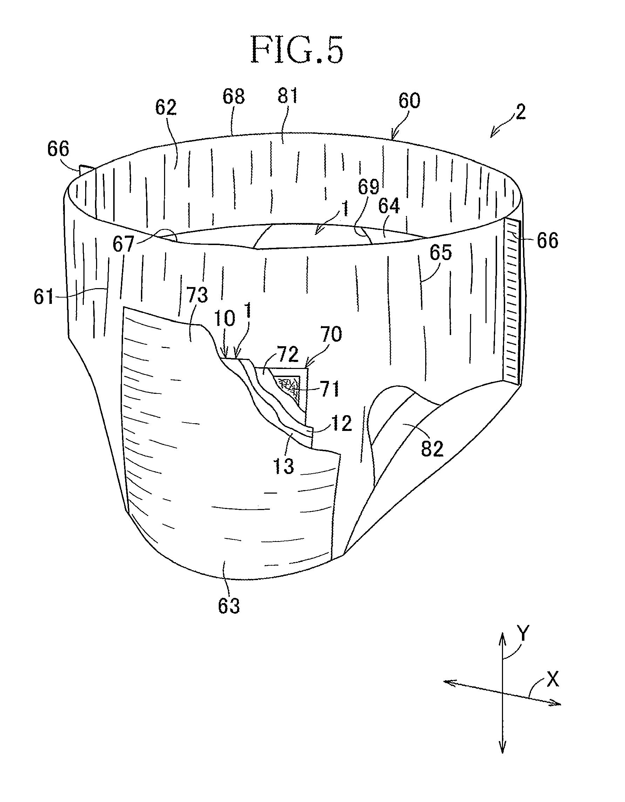

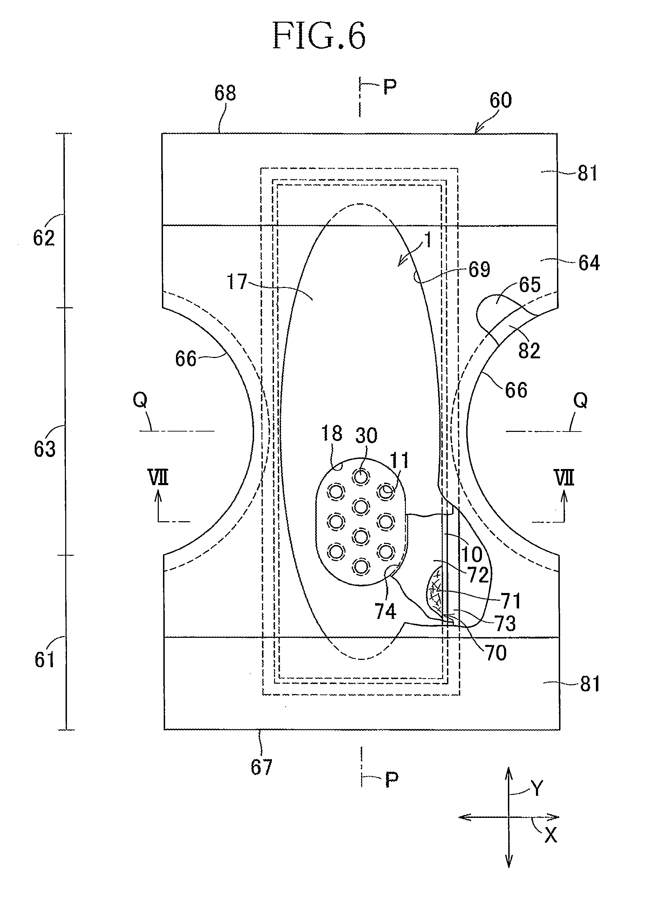

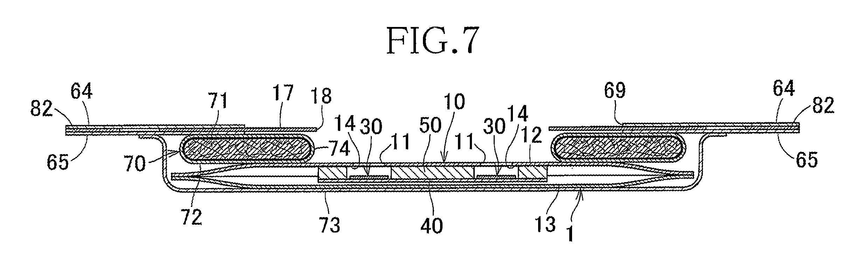

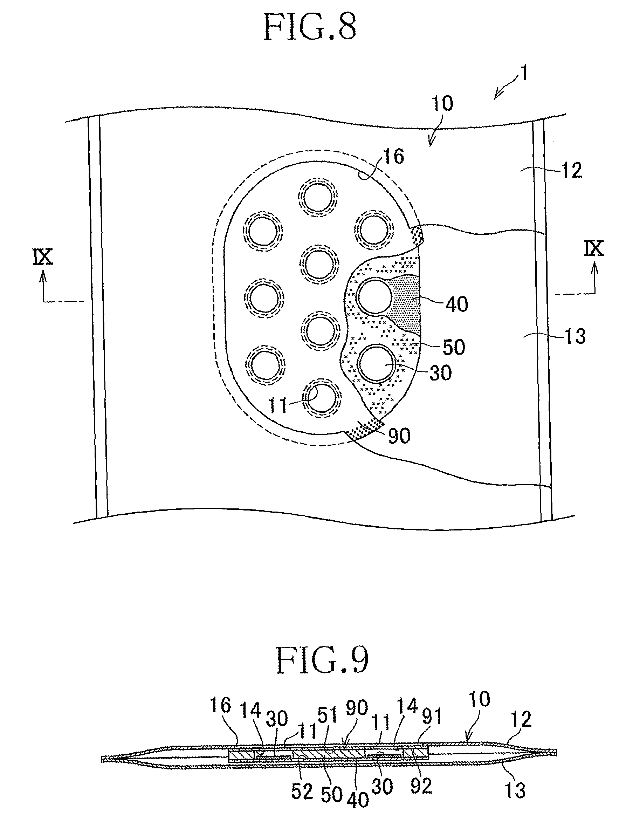

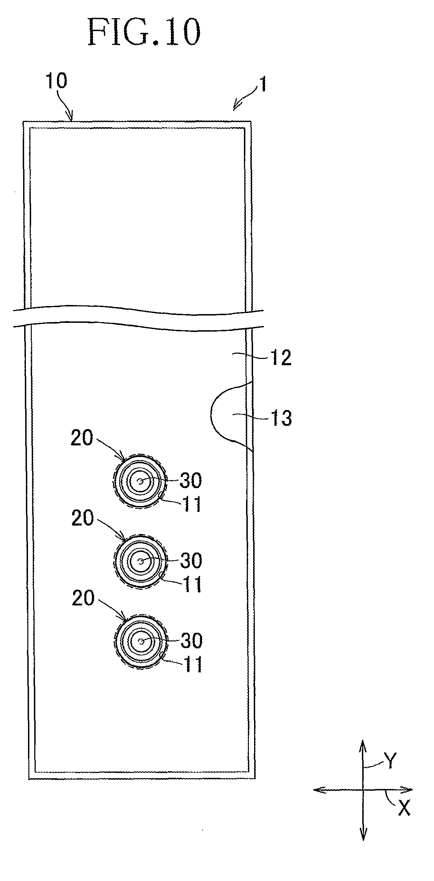

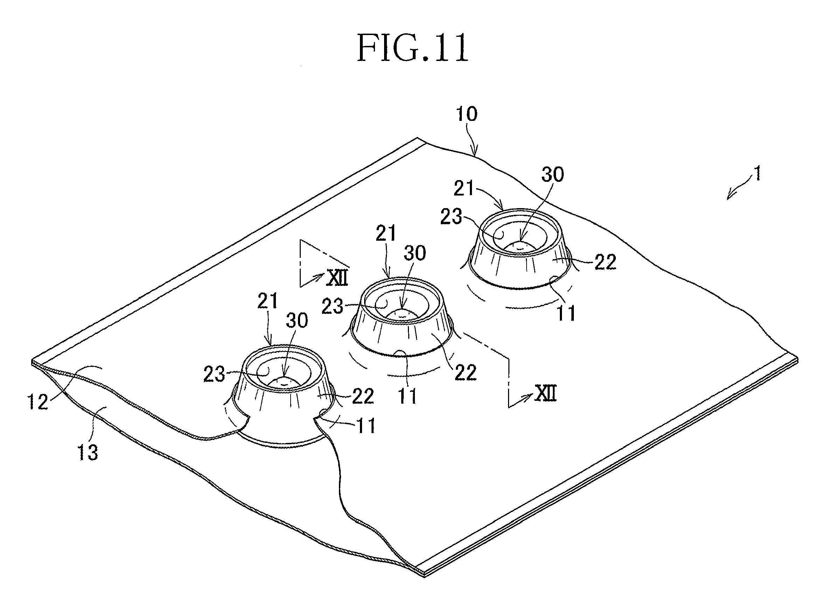

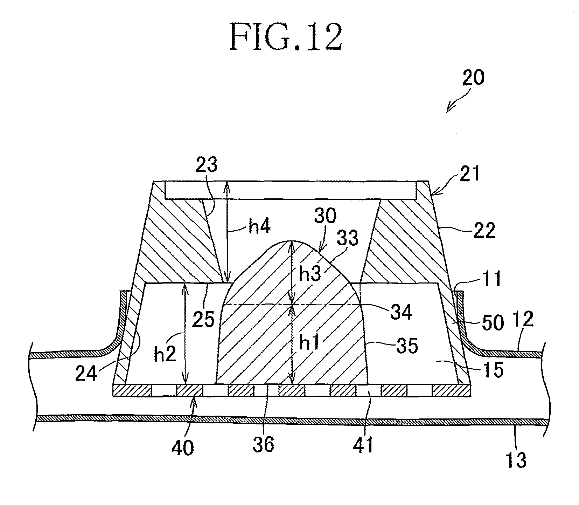

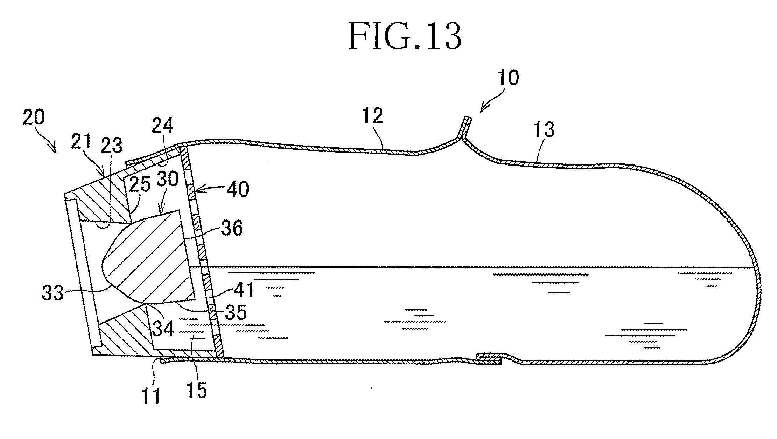

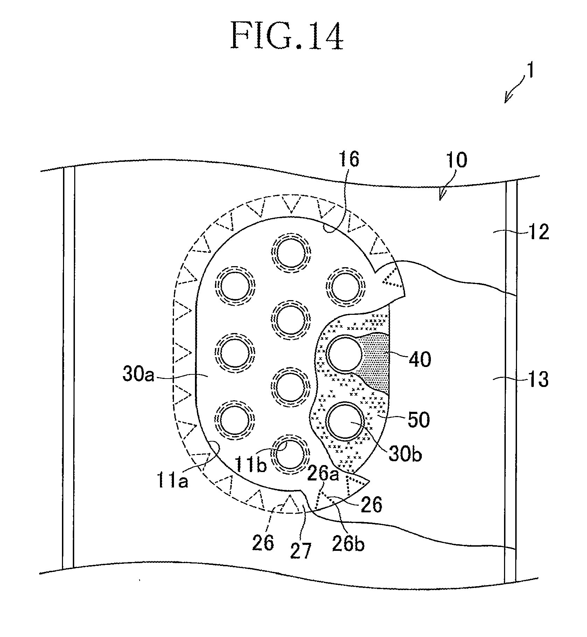

{Fig. 1} A plan view of a bodily fluid trapping unit according to a first exemplary embodiment.
{Fig. 2} A schematic sectional view taken along the line II-II in Fig. 1.
{Fig. 3} A perspective view showing a part of Fig. 1.
{Fig. 4} A diagram illustrating the bodily fluid trapping unit. {Fig. 5} A perspective view of a diaper.
{Fig. 6} A plan view showing the diaper in its flatly developed state.
{Fig. 7} A schematic sectional view taken along the line VII-VII in Fig. 6.
{Fig. 8} A partial plan view of a bodily fluid trapping unit according a second exemplary embodiment.
{Fig. 9} A sectional view taken along the line IX-IX in Fig. 8.
{Fig. 10} A plan view of a bodily fluid trapping unit according to a third exemplary embodiment.
{Fig. 11} A perspective view showing a part of Fig. 10.
{Fig. 12} A schematic sectional view taken along the line XII-XII in Fig. 11.
{Fig. 13} A diagram illustrating the bodily fluid trapping unit.
{Fig. 14} A partial plan view of a bodily fluid trapping unit according to a fourth exemplary embodiment.

### {Description of Embodiments}

### {Embodiment 1}

Figs. 1 through 4 illustrate the first exemplary embodiment of a bodily fluid trapping unit according to the present invention wherein Fig. 1 is a plan view of a bodily fluid trapping unit 1, Fig. 2 is a schematic sectional view taken along the line II-II in Fig. 1, Fig. 3 is a perspective view showing a part of Fig. 1 and Fig. 4 is a diagram illustrating the bodily fluid trapping unit 1. Figs. 1 and 3 are partially cutaway for convenience of illustration. As shown, the bodily fluid trapping unit 1 has a longitudinal direction Y and a transverse direction X and includes a liquid-impervious receptacle 10 formed with a plurality of openings 11 and backflow preventing means 20 provided at positions corresponding to the respective openings 11.

The receptacle 10 is defined by a first sheet 12 lying on the side thereof facing the wearer's skin cooperating with a second sheet 13 lying on the side thereof facing the wearer' garments, both sheets 12, 13 being bonded together along respective peripheral edges thereof to form the bag-shaped receptacle 10. The first sheet 12 is formed with a plurality of the annularly cut out openings 11 each extending through the first sheet 12 in a thickness direction thereof so that bodily fluid such as urine may inflow. These openings 11 are arranged so that each pair of the adjacent openings 11 may be appropriately spaced from each other in the longitudinal direction Y as well as in the transverse direction X. As stock material for these first and second sheets 12, 13, a liquid-impervious sheet, for example, a laminate of a plastic film and a fibrous non-woven fabric or a melt blown fibrous non-woven fabric made from micro-fibers and having a relatively high density may be used.

The backflow preventing means 20 are provided in association with the respective openings 11 and includes flat valve discs 30 each having a specific gravity lower than that of bodily fluid such as urine and arranged on the side of the first sheet 12 facing the receptacle 10, a sheet-like support base 40 serving to prevent the valve discs 30 from falling into the receptacle 10 and a spacer 50 sandwiched between the first sheet 12 and the support base 40.

The valve disc 30 is formed, for example, of a fibrous panel, a foamed plastic or a chartaceous material which each having a stiffness higher than that of the receptacle and the support base 40 and having a high water resistance. The valve disc 30 has an upper surface 31 facing the first sheet 12 and a lower surface 32 facing the second sheet 13. A plurality of the valve discs 30 are provided associated with a plurality of the openings 11 and the individual valve disc 30 has an area larger than an opening area of the individual opening 11 so that a peripheral edge of the valve disc's upper surface 31 may come in water-tight contact with a valve seat 14 formed on the side of the opening 11 facing the receptacle 10 along a peripheral edge of the opening 11. While the opening 11 has a diameter of about 6mm and the valve disc 30 has a diameter of about 12. 5mm in this embodiment, the invention is not limited to these particular dimensions and may be appropriately varied depending on various factors such as the number and positions of the opening 11 and a size of the bodily fluid trapping unit 1.

Between the first sheet 12 and the support base 40, a spacer 50 is interposed. The spacer 50 may be formed using stock material having a relatively high shape retaining property, for example, a fibrous panel, a foamed plastic or a chartaceous material. The spacer 50 is substantially formed of ellipse having an area sufficiently large to enclose a plurality of openings 11 as a whole and defined by a major axis being coincident with the longitudinal direction Y and a minor axis being coincident with the transverse direction X. The spacer 50 has an upper surface 51 facing the first sheet 12 and a lower surface 52 facing the second sheet 13 wherein the upper surface 51 is bonded to the first sheet 12 and the lower surface 52 is bonded to the support base 40 by appropriate means such as adhesive. The spacer 50 is formed with spacer openings 53 extending through the spacer 50 from the upper surface 51 to the lower surface 52 in alignment with the associated openings 11 and these spacer openings 53 are respectively provided with the valve discs 30. As stock material for the support base 40, for example, a liquid-pervious fibrous non-woven fabric may be used.

The spacers' openings 53 respectively define spaces 15 between the first sheet 12 and the support base 40. The valve disc 30 is supported within the associated space 15 so as to be movable in the thickness direction of the spacer 50. Specifically, the spacer's opening 53 has a diameter larger than a diameter of the opening 11 as well as a diameter of the valve disc 30. A thickness dimension of the spacer 50 is larger than a thickness dimension of the valve disc 30. More specifically, the valve disc 30 has a thickness of about 0.5mm and the spacer 50 has a thickness of about 2mm.
The support base 40 is bonded to the spacer's lower surface 52 and therefore the valve disc 30 should not fall into the receptacle 10. In addition, a peripheral wall of the spacer 50, the valve seat 14 and the support base 40 cooperated together to restrict a movement of the valve disc 30 in both the longitudinal direction Y and the transverse direction X.

When bodily fluid such as urine is discharged into the openings 11 of such bodily fluid trapping unit 1, a weight of bodily fluid causes the valve discs 30 to move down toward the support base 40 and the valve discs' lower surfaces 32 are held in contact with the support base 40. The valve discs' upper surfaces 31 are spaced downward from the associated valve seats 14 and thus bodily fluid flows through clearances defined around peripheries of the respective valve discs 30 into the associated spaces 16. From these spaces 16, bodily fluid further flows through the support base 40 into the receptacle 10. The valve disc 30 can be spaced from the associated valve seat 14 along the entire periphery and thereby allows bodily fluid to flow into the associated space 15 through the circumferential clearance. As a result, bodily fluid can quickly flow into the space 15 from any direction and therefore it is not apprehended that any amount of bodily fluid might stay outside the receptacle 10. The receptacle 10 is provided in the form of a liquid-impervious bag adapted to contain bodily fluid therein.

If the receptacle 10 containing bodily fluid therein is inverted upside down as shown in Fig. 4, bodily fluid moves toward the openings 11 so that the lower surface 32 of the valve disc 30 is forced by bodily fluid having passed through the support base 40 to move toward the first sheet 12 until the upper surface 31 of the valve disc 30 comes in contact with the associated valve seat 14. The valve disc 30 can be kept in water-tight contact with the valve seat under a pressure of bodily fluid and, in this way, bodily fluid should not flow back from the receptacle 10.
In addition, the valve disc 30 has a specific gravity sufficiently lower than that of bodily fluid to float on bodily fluid. With the receptacle 10 being in the posture as shown in Fig. 2 and containing therein bodily fluid such as urine, the openings 11 are sealed by the associated valve discs 30 before bodily fluid may flow out through the openings 11 since the valve discs 30 float on bodily fluid. In such case also, bodily fluid should not flow out from the receptacle 10.

Even if slight amount of bodily fluid stays behind between the valve discs 30 and the first sheet 12, surface tension of bodily fluid functions to enhance water-tight contact between the valve disc's upper surface 31 and the associated valve seat 14 defined by the first sheet 12 and thereby to improve a preventive effect against backflow of bodily fluid from the receptacle 10.
For the reason that a plurality of the openings 11 as well as the backflow preventing means 20 are formed, it is possible to avoid a situation that the entire area of the backflow preventing means 20 might cease to function properly even if one of the valve discs 30 is immobilized in the thickness direction. The area occupied by these openings 11 and the backflow preventing means 20 may be enlarged, if it is desired, to collect and to contain bodily fluid in the receptacle further reliably.

Figs. 5 through 7 exemplarily illustrate a wearing article including the bodily fluid trapping unit 1 as has been described above and a disposable diaper 2 will be described as a typical embodiment of such wearing articles.
Fig. 5 is a perspective view of the diaper 2, Fig. 6 is a plan view showing the diaper 2 having front and rear waist regions disconnected from each other along side edges thereof from the state shown in Fig. 5 and flatly developed and Fig. 7 is a sectional view taken along the line VII-VII in Fig. 6. Figs. 5 and 6 illustrate the diaper 2 as partially cutaway for convenience of illustration and Fig. 6 illustrates the diaper 2 as flatly developed by stretching respective elastic members against contractile force thereof. The diaper 2 basically includes a chassis 60 and a liquid-absorbent structure 70. The chassis 60 includes a front waist region 61, a rear waist region 62 and a crotch region 63 extending between these front and rear waist regions 61, 62, these regions being continuous as viewed in the longitudinal direction Y. The chassis 60 has a longitudinal imaginary center line P-P bisecting a dimension of the chassis 60 in the transverse direction X and a transverse imaginary center line Q-Q bisecting a dimension of the chassis 60 in the longitudinal direction Y.

The chassis 60 includes a liner sheet 64 defining the side thereof facing the wearer's skin, an outer sheet 65 defining the side thereof facing the wearer's clothes, side edges 66, 66 extending in the longitudinal direction Y and front and rear ends 67, 68 extending in the transverse direction X. The side edges 66, 66 concavely curve in the crotch region 63 toward the longitudinal imaginary center line P-P. The liner sheet 64 and the outer sheet 65 may be formed using material widely used in the relevant technical field such as liquid-pervious fibrous non-woven fabrics.
At least in the crotch region 63, leg elastic elements 82 are attached under tension to the chassis 60 along the side edges 66, 66 thereof so as to be contractible. These leg elastic elements 82 are sandwiched between the liner sheet 64 and the outer sheet 65 and bonded to at least one of these sheets 64, 65.

Waist elastic elements 81, 81 each having a relatively large width dimension are attached under tension in the transverse direction X to the chassis 60 along the front and rear ends 67, 68 thereof so as to be contractible. Specifically, these waist elastic elements 81, 81 are bonded to the side of the liner sheet 64 facing the wearer's skin. Both the waist elastic elements 81 and the leg elastic elements 82 may be formed by elasticized fibrous non-woven fabric but are not limited to this and may be formed by the other elasticized material conventionally used in the relevant technical field. As illustrated in Fig. 5, the front and rear waist regions 61, 62 are joined together along the chassis's side edges 66, 66 to form the pants-type diaper 2.

The chassis 60 is formed, at least in the crotch region 63, with a chassis's opening 69 extending through the liner sheet 64 and the outer sheet 65 in the thickness direction. According to this embodiment, the chassis's opening 69 is provided substantially in an elliptical shape extending across the crotch region 63 into the front and rear waist regions 61, 62. Between these liner sheet 64 and outer sheet 65, a top-sheet 17 is interposed so as to close up the chassis's opening 69. The top-sheet 17 is formed in a region defined aside from the transverse imaginary center line Q-Q toward the chassis's front end 67 with a substantially elliptical top-sheet's opening 18 extending through the top-sheet 17 in the thickness direction and has an opening area smaller than that of the chassis's opening 69.

The outer sheet 65 is provided on its garment-facing side with the liquid-absorbent structure 70 so as to close up the chassis's opening 69, i.e., to overlie the top-sheet 17. The liquid-absorbent structure 70 has a skin-facing side, a garment-facing side, a liquid-absorbent core 71 and a wrapping sheet 72 adapted to wrap the liquid-absorbent core 71. The liquid-absorbent structure 71 is placed aside from the transverse imaginary center line Q-Q toward the chassis's front end 67 and formed with an opening 74 corresponding to the top-sheet's opening 18. The liquid-absorbent structure's opening 74 extends through the liquid absorbent core 71 in the thickness direction. The liquid-absorbent core 71 may be formed, for example, of a mixture of fluff pulp fibers and super-absorbent polymer particles and, as stock material for the wrapping sheet 72, for example, a liquid-dispersant tissue paper may be used. Both the liquid-absorbent core 71 and the wrapping sheet 72 may be formed also of the other materials conventionally used in the relevant technical field.
The liquid-absorbent structure 70 is provided on its skin-facing side with the bodily fluid trapping unit 1 which is, in turn, provided on its garment-facing side with a cover sheet 73. As stock material for the cover sheet 73, for example, a liquid-impervious film may be used. It is also possible to provide the cover sheet 73 on its garment-facing side with a fibrous non-woven fabric and thereby to provide a good feeling of the article against the wearer's skin.

The bodily fluid trapping unit 1 is sandwiched between the liquid-absorbent structure 70 and the cover sheet 73 in a manner that the first sheet 1 thereof lies on the side of the liquid-absorbent structure 70 and the second sheet 13 lies on the side of the cover sheet 73. The openings 11 of the bodily fluid trapping unit 1 are arranged aside from the transverse imaginary center line Q-Q toward the chassis's front end 67 and exposed on the skin-facing side by the intermediary of the liquid-absorbent structure's opening 74 and the top-sheet's opening 18. The region occupied by the openings 11 is located so as to face the wearer's external genital.

With the diaper 2 as has been outlined above, upon urination, urine discharged by the wearer flows through the openings 11 into the receptacle 10 of the bodily fluid trapping unit 1. When the bodily fluid trapping unit 1 held in the posture as shown in Fig. 2, urine flows through the circumferential clearance defined around the valve disc 30 into the receptacle 10. When the bodily fluid trapping unit 1 takes the posture of Fig. 4 as the wearer lies down or gets down on all fours, the valve disc 30 comes in water-tight contact with the associated valve seat 14 of the first sheet 12 so as to prevent urine once having been collected into the receptacle 10 from counter-flowing toward the side of the wearer's skin. When the wearer's body weight is exerted upon the bodily fluid trapping unit 1, for example, as the wearer gets seated, urine' s level within the receptacle 10 rises. However, the valve disc 30 has a specific gravity sufficiently lower than that of bodily fluid such as urine to float on urine and, as a consequence, the upper surface 21 of the valve disc 30 comes in contact with the associated valve seat 14 of the first sheet 12 before urine might flow out from the receptacle 10. Therefore, it is possible in such a case also to prevent urine from flowing back toward the side of the wearer's skin. In this way, it is assured to prevent the back-flow of urine from the bodily fluid trapping unit 1 irrespectively of the unit posture and eventually it is assured to restrict urine leakage from the diaper 2.

Even if urine is discharged onto a region off from the region occupied by the openings 11 or any amount of urine leaks out from the receptacle 10, such amount of urine can be absorbed by the liquid-absorbent structure 70 which is present around the region of the openings 11. With such an arrangement, it is further assured to restrict urine leakage from the diaper 2. The space 15 serving to contain the valve disc 30 is defined by the spacer 50 and a thickness dimension of the spacer 50 can be reduced by utilizing the disc-like valve to alleviate an uncomfortable feeling created against the wearer.

While the liquid-absorbent structure 70 of the diaper 2 is formed with the opening 74 by the intermediary of which the openings 11 of the bodily fluid trapping unit 1 are exposed according to this embodiment, it is possible without departing from the scope of the invention to dispose the bodily fluid trapping unit 1 as a whole on the side of the liquid-absorbent structure 70 facing the wearer's skin so that the bodily fluid trapping unit 1 may be sandwiched between the chassis 60 and the liquid-absorbent structure 70.

While the chassis 60 is formed with the opening 69 according to this embodiment, it is not essential for the invention to form the chassis 60 with such opening 69 so far as at least the region of the chassis corresponding to the region occupied by the openings 11 of the bodily fluid trapping unit 1 are liquid-pervious so that bodily fluids such as urine may permeate this region of the chassis 60 into the bodily fluid trapping unit 1 disposed on the garment-facing side of the chassis 60. It is also possible to dispose the bodily fluid trapping unit 1 on the skin-facing side of the chassis 60. Whatever the case may be, it is essential that bodily fluids such as urine can be collected through the openings 11 of the bodily fluid trapping unit 1 into the receptacle 10. While the receptacle 10 is formed of the liquid-impervious material according to this embodiment, the stock material for the receptacle 10 is not limited to the liquid-impervious material. Specifically, it is possible to use a liquid-pervious material as the stock material for the receptacle 10 so far as bodily fluids flowing through the openings 11 can be collected and contained, for example, by the liquid-absorbent core. In this regard, on the assumption that the liquid-pervious sheet is used as the first sheet 12 and the respective valve seats 14 are defined by the peripheries of the associated openings 11 formed through the first sheet 12, at least the valve seats 14 must be liquid-impervious to assure that the valve discs 30 can come in water-tight contact with the associated valve seats 14. For example, in the case of the first sheet 12 formed of a liquid-pervious fibrous non-woven fabric, the valve seats 14 may be formed by bonding a liquid-impervious film to the lower surface around the respective openings 11 and in the case of the first sheet 12 formed of a hydrophobic fibrous non-woven fabric treated to become hydrophilic, the valve seats 14 may be formed by excluding the regions of the first sheet 12 adapted to define the valve seats 14 from such treatment.

### [Embodiment 2]

Figs. 8 and 9 illustrate the second exemplary embodiment of the invention wherein Fig. 8 is a plan view of the bodily fluid trapping unit 1 as partially cutaway for convenience of illustration and Fig. 9 is a sectional view taken along the line IX-IX in Fig. 8. This first embodiment of the bodily fluid trapping unit 1 is **characterized in that** the first sheet 12 itself is not formed with the openings 11 but a shape-containing member 90 formed with the openings 11 are separately prepared. The other features are similar to those of the first exemplary embodiment and details thereof will not be described.

The first sheet 12 constituting the receptacle 10 is formed with an attachment opening 16 for the shape-retaining member 90. The shape-retaining member 90 formed with the openings 11 is bonded to the side of the attachment opening 16 facing the second sheet 13 along a peripheral edge of the attachment opening 16 by appropriate joining means such as adhesive. The shape-retaining member 90 may be formed by material having relatively high shape retention such as a fibrous panel, a foamed plastic or a chartaceous material and has a stiffness higher than that of the first sheet 12. The shape-retaining member 90 has an upper surface 91 lying on the side of the first sheet 12 and a lower surface 92 lying on the side of the second sheet 13 wherein the upper surface 91 is bonded to he peripheral edge of the attachment opening 16 of the first sheet 12 and the lower surface 92 is bonded to the upper surface 51 of the spacer 50.

By forming the shape-retaining member 90 with the openings 11 and then bonding this shape-retaining member 90 to the first sheet 12, the shapes of the respective openings 11 can be more reliably retained than the case in which the first sheet 12 is directly formed with these openings 11. If the openings 11 get distorted and lose initial shapes thereof, it will be difficult for the valve discs 30 to come in close contact with the associated valve seats 14 and, in consequence, undesirable clearances should be left. Such clearances should cause leakage of bodily fluids such as urine. However, by retaining the shapes of the openings 11, the valve discs 30 can be reliably put in water-tight contact with the associated valve seats 14 to prevent leakage of bodily fluids. According to this embodiment, the valve seats 14 are formed on the lower surface 91 of the shape-retaining member 90.

The procedure of forming the shape-retaining member 90 with the openings 11 and then bonding this to the first sheet 12 facilitates the openings 11 to be formed in comparison with the case in which the first sheet 12 itself is formed with the openings 11. This is because the shape-retaining member 90 has an area smaller than that of the first sheet 12 and a stiffness higher than that of the first sheet 12. While the shape-retaining member 90 and the spacer 50 are provided as an independent member in this embodiment, these members may be formed by a single member.
While the shape-retaining member 90 is attached to the side of the first sheet 12 facing the second sheet 13, i.e., facing the receptacle 10 in this embodiment, it is possible to attach the shape-retaining member 90 to the side of the first sheet 12 facing away from the receptacle 10. Such bodily fluid trapping unit 1 may be, as the unit of the first embodiment, used in the wearing article such as the diaper 2.

### {Embodiment 3}

Figs. 10 through 13 illustrate the third exemplary embodiment of the invention wherein Fig. 10 is a plan view of a bodily fluid trapping unit as a whole, Fig. 11 is a perspective view showing a part of Fig. 10, Fig. 12 is a schematic sectional view taken along the line XII-XII in Fig. 11 and Fig. 13 is a diagram illustrating the bodily fluid trapping unit. As illustrated, the bodily fluid trapping unit 1 includes the liquid-impervious receptacle 10, a plurality of the openings 11 form in the receptacle 10 and the backflow preventing means 20 formed in the respective openings 11. This embodiment is similar to the first embodiment except a construction of the backflow preventing means 20 and the features similar to those in the first embodiment will not be repetitively described.

The backflow preventing means 20 includes the valve seat body 21 and the valve disc 30. The valve seat body 21 is attached along the opening 11 and more specifically an outer peripheral surface 22 of the valve seat body 21 is bonded to the first sheet 12. The valve seat body 21 is formed substantially at a center thereof with a first valve port 23 and a second valve port 24 extending through a center of the valve seat body 21 in the thickness direction. The first valve port 23 has a diameter smaller than that of the second valve port 24 and the second valve port 24 is closer to the second sheet 13 than the first valve port 23 is. A differential diameter between the first valve port 23 and the second valve port 24 forms a step which defines, in turn, a valve seat 25. The second valve port 24 defines a peripheral wall extending toward the receptacle 10 serving as the spacer 50 which defines, in turn, the space 15.

The support base 40 is bonded to the side of the second valve port 24 facing the second sheet 13 by means of adhesive or the like so as to cover the second valve port 24. The support base 40 is liquid-pervious. More specifically, the support base 40 is formed by a resinous plate formed with a plurality of liquid-pervious pores 41 to make the support base 40 liquid-pervious. The support base 40 is not limited to such liquid-pervious resinous plate and, for example, it is possible to use, for example, liquid-pervious non-woven fabric as the support base 40.

Within the space 15, a valve plug 30 is movably supported in the thickness direction. The valve plug 30 used in this embodiment has a columnar shape and extending upward from the second valve port 24 toward the first valve port 23. More specifically, the valve plug 30 includes a small diameter section 33 normally staying within the first valve port 23 and having a diameter smaller than that of the valve seat 25, a large diameter section 35 having a diameter larger than that of the valve seat 25 and normally staying within the second valve port 24, i.e., positioned aside from the small diameter section 33 toward the receptacle 10 and a contact section 34 defined between these small diameter section 33 and the large diameter section 35 and adapted to come in contact with the valve seat 25. The contact section 34 is able to come in water-tight contact with the valve seat 25. To assure the contact section 34 to come in water-tight contact with the valve seat 25, the valve plug 30 is preferably formed, for example, by flexible resin such as polyurethane. Such valve plug 30 has a flat bottom 36 positioned on the side facing the receptacle 10.

The valve plug 30 as has been described just above is formed by the material having a specific gravity lower than that of bodily fluids so that the valve plug 30 may float on bodily fluids when the receptacle 10 is filled with bodily fluids. A height dimension h1 from the contact section 34 to the bottom 36 of the valve plug 30 is smaller than a height dimension h2 of the spacer 50 defined by the second valve port 24, i.e., a height dimension from the valve seat 25 to the support base 40. With such dimensional relationship, the contact section 34 of the valve plug 30 is spaced from the valve seat 25 and the bottom 36 of the valve plug 30 rests on the support base 40 when the valve plug 30 is positioned above the support base 40 as illustrated by Fig. 12.

The valve seat body 21 is preferably formed of, for example, resinous material having a sufficiently high degree of hardness to retain the shape of the opening 11. As used herein, the term "sufficiently high degree of hardness to retain the shape of the opening 11" means the degree of hardness assuring that at least the first and second valve ports 23, 24 can be shape-retained even when a body weight of the wearer is exerted on the valve seat body 21. By shape-retaining the first and second valve ports 23, 24, it is assured that the valve plug 30 comes in water-tight contact with the valve seat 25 substantially without leaving a clearance therebetween.

Assuming that the bodily fluid trapping unit 1 as has been described just above is kept in a posture as illustrated by Fig. 12, the valve plug 30 is spaced from the valve seat 25 and bodily fluid such as urine discharged into the first valve port 23 is guided to pass through the clearance around the small diameter section 33 of the valve plug 30 and to flow into the receptacle 10 through the liquid-pervious pores 41 of the support base 40.

Assuming that the posture of the bodily fluid trapping unit 1 has been changed from the posture as shown in Fig. 12 to the posture as shown in Fig. 13, bodily fluid counterflows through the liquid-pervious pores 41 of the support base 40 to the bottom 36 of the valve plug 30 and pushes this valve plug 30 toward the side facing away from the receptacle 10. The valve plug 30 has a specific gravity sufficiently lower than bodily fluid to float on bodily fluid and thereby the valve plug 30 is further pushed toward the side facing away from the receptacle 10 until the contact section 34 of the valve plug 30 comes in contact with the valve seat 25. Appropriate flexibility of the valve plug 30 allows the valve plug 30 to come in water-tight contact with the valve seat 25 and thereby to prevent back-flow of bodily fluid from the receptacle 10.
Even when the posture of the receptacle 10 is reversed, the bottom 36 of the valve plug 30 is pushed by urine and, in addition, the receptacle's own weight forces the contact section 34 of the valve plug 30 in water-tight contact with the valve seat 25. Consequentially, bodily fluid should note flow out from the receptacle.

In the bodily fluid trapping unit 1 as has been described just above, a height dimension h3 from the tip of the small diameter section 33 to the contact section 34 is preferably smaller than a height dimension h4 of the first valve port 23 as illustrated by Fig. 12. Such dimensional relationship eliminates the apprehension that the tip of the valve plug 30 might project beyond the first valve port 23, come in contact with the wearer's skin and cause any kind of skin trouble. Even if the valve plug 30 comes in contact with the wearer's skin, impact due to this contact can be alleviated since the valve plug 30 is made of flexible resin.
Such bodily fluid trapping unit 1 may be usefully incorporated into the wearing article as has been described with respect to the first exemplary embodiment.

While the valve seat body 21 and the support base 40 are formed as separate members and then bonded together according to this third embodiment, it is possible without departing from the scope of the invention to form them as an integrated member made of one and same type of material.

### {Embodiment 4}

Fig. 14 is a plan view of the bodily fluid trapping unit 1 according to the fourth exemplary embodiment of the invention as partially cutaway for convenience of illustration. The bodily fluid trapping unit 1 according to this fourth embodiment is **characterized in that** a plurality of openings having different sizes are formed and two or more types of backflow preventing means having different modes of action are provided. The other features are similar to those of the embodiments 1 and 2 and details thereof will not be described.

The first sheet 12 constituting the receptacle 10 is formed with a first opening 11a and a first backflow preventing means associated with the first opening 11a. The first backflow preventing means includes a sheet-like valve disc 30a formed, for example, of a plastic film and opposed to and attached to the first opening 11a and bonding regions 26 in which the valve disc 30a is bonded to the first sheet 12. The valve disc 30a and the first sheet 12 may be heat-sealed to each other under heating and pressurizing to define the respective bonding regions 26. It is also possible to use appropriate adhesive such as hot melt adhesive to define the bonding regions 26.

The bonding regions 26 are regions in which the valve disc 30a and the first sheet 12 are laminated together. Each of the bonding regions 26 linearly extends outward from a peripheral edge of the first opening 11a and has a V-shape. More specifically, a pair of adjacent bonding lines comes in contact with each other at inner ends 26 of the bonding region 26 and these two adjacent bonding lines are spaced from each other at outer ends 26b of the bonding region 26. Between a pair of the adjacent bonding regions 26, 26, a channel 27 allowing bodily fluid to flow into the receptacle 10 is defined. While the inner ends 26a of these two adjacent bonding lines come in contact with each other in the illustrated embodiment, it is not essential that these two inner ends come in contact with each other. Alternatively, it is possible to form the bonding region 26 wherein the two adjacent inner ends are spaced from each other. In such alternative arrangement also, the two adjacent bonding lines preferably extend obliquely so that the respective inner ends 26a may come close to each other.

The valve disc 30a is formed with a plurality of second openings 11b and second backflow preventing means associated with these second openings 11b. Each of the second backflow preventing means associated with the second openings 11b includes flat valve disc 30b provided on the side of the first sheet 12 facing the receptacle 10 and having a specific gravity lower than that of bodily fluid such as urine, a sheet-like support base 40 and a spacer 50 interposed between the first sheet 12 and the support base 40. The valve disc 30b is adapted to come in contact with the inner surface of the valve disc 30a and thereby to close the associated opening 11b. The support base 40 and the spacer 50 are similar to those in the first embodiment.

Bodily fluid such as urine discharged onto such bodily fluid trapping unit 1 can flow into the receptacle 10 through the first opening 11a and the second openings 11b. Specifically, in the first opening 11a, the first sheet 12 and the valve disc 30a are spaced from each other under the effect of bodily fluid weight. A plurality of channels 27 are defined between a pair of the adjacent bonding regions 26. The bodily fluid may flow through the channels 27 into the receptacle 10. The channels 27 are defined along a periphery of the first opening 11a. Therefore, the bodily fluid can flow into the receptacle 10.

In the second openings 11b, the valve discs 30b are depressed toward the support base 40 under the effect of bodily fluid weight so as to be spaced from the valve disc 30a so that bodily fluid may flow through clearances defined around the respective valve discs 30b into the receptacle 10. Even if bodily fluid is not fully received by the second openings 11b and some amount of bodily fluid stays on the first valve disc 30a, the first opening 11a surrounding these second openings 11b allows such amount of bodily fluid to flow into the receptacle 10. By forming these different sized openings 11a, 11b in this manner, bodily fluid can be reliably guided into the receptacle 10.

Assuming that the receptacle 10 retaining therein bodily fluid is reversed, bodily fluid moves toward the openings 11a, 11b and simultaneously the second valve discs 30b are depressed by bodily fluid having permeated the support base 40 to move toward the first valve disc 30a until these valve discs 30b come in water-tight contact with the valve disc 30a and thereby prevent outflow of bodily fluid from the receptacle 10.

There is no possibility that bodily fluid might counterflow outward from the receptacle 10 through the first opening 11a because the outer ends 26b of the bonding regions 26 effectively narrow the respective channels 27. In addition, a slight amount of bodily fluid staying between the valve disc 30a and the first sheet 12 assists these two sheets to be kept in water-tight contact and thereby the channels to be constricted. In this way, leakage of bodily fluid can be further reliably prevented. Two or more types of backflow preventing means which are different in size as well as in mode of action may be provided to obtain two or more types of backflow preventing effects peculiar to the respective types of these means.

When two or more types of backflow preventing means are provided, the arrangement and combination are not limited to those according to the fourth exemplary embodiment as has been described above and the other various combinations are available. While the openings of different sizes are provided in this embodiment, the combination of these openings depends on the backflow preventing means to be selected.

### {Reference Signs List}

- 1: bodily fluid trapping unit
- 2: diaper (wearing article)
- 10: receptacle
- 11: openings
- 11a: opening
- 11b: openings
- 14: valve seats
- 15: spaces
- 20: backflow preventing means
- 25: valve seats
- 26: bonding regions
- 27: channels
- 30: valve discs (or valve plugs)
- 30a: valve discs
- 30b: valve discs
- 33: small-diameter portion
- 34: contact region
- 35: large-diameter portion
- 40: support base
- 50: spacer
- 60: chassis
- 61: front waist region
- 62: rear waist region
- 63: crotch region
- 69: chassis opening
- 70: liquid-absorbent structure
- 74: liquid-absorbent opening

## Claims

1. A bodily fluid trapping unit which comprises openings through which bodily fluid flows in and a receptacle adapted to collect bodily fluid flowing thereinto being **characterized in that**:
the bodily fluid trapping unit comprises backflow preventing means adapted to restrict outflow of bodily fluid once having been collected in the receptacle through the openings and a plurality of the openings penetrating a part of the receptacle in a thickness direction; and
the backflow preventing means comprises a valve seat formed along a peripheral edge of the openings, a valve disc adapted to come in water-tight contact with the valve seat from the side of the receptacle and a support base adapted to prevent the valve disc from falling into the receptacle wherein the bodily fluid is allowed to flow into the receptacle as the valve disc is spaced from the valve seat.

2. The bodily fluid trapping unit defined by Claim 1, wherein a spacer is interposed between the valve seat and the support base to maintain a space and the valve disc is movably supported within the space.

3. The bodily fluid trapping unit defined by Claim 1 or 2 wherein
the valve disc is flat and has an area larger than an opening area of each of the opening.

4. The bodily fluid trapping unit defined by Claim 1 or 2, wherein:
the valve seat is defined by a valve seat body formed with a valve port extending through the valve seat body in its thickness direction; and
the valve seat body includes a small diameter section having a diameter smaller than that of the valve port, a large diameter section having a diameter larger than that of the valve port and a contact section defined between the small diameter section and the large diameter section and adapted to come in water-tight contact with the valve port wherein the small diameter section lies on the side facing away from the receptacle and the large diameter section lies on the side facing the receptacle.

5. The bodily fluid trapping unit defined by any one of Claims 1 through 4, wherein the valve disc has a specific gravity lower than that of the bodily fluid.

6. The bodily fluid trapping unit defined by Claim 1, wherein the backflow preventing means comprises a valve disc provided so as to overlap along the periphery of the opening and bonding regions in which the valve disc is bonded to the receptacle, each of the bonding regions is defined by a pair of bonding lines extending outward from the opening so that each pair of the adjacent bonding regions defines a channel allowing bodily fluid to flow into the receptacle through the opening.

7. The bodily fluid trapping unit defined by any one of Claims 1 through 6, wherein two or more types of the backflow preventing means having different modes of action.

8. A wearing article having a longitudinal direction, a transverse direction, a skin-facing side and a garment-facing side and comprising a front waist region, a rear waist region and a crotch region extending between these front and rear waist regions so that these regions may be contiguous one to another in the longitudinal direction, the wearing article being **characterized in that**:
the wearing article includes the bodily fluid trapping unit defined by any one of Claims 1 through 7 at least in the crotch region; and
the opening of the bodily fluid trapping unit is formed on the skin-facing side.
